# EUROPÄISCHE PATENTSCHRIFT

(11) **EP 1 003 718 B1**
(45) Veröffentlichungstag und Bekanntmachung des Hinweises auf die Patenterteilung: **28.04.2004**
(21) Anmeldenummer: 98940212.8
(22) Anmeldetag: 13.07.1998
(51) Int. Cl.: C07C 303/38, C07C 303/40, C07C 211/52, C07C 311/08, C07C 311/48

(54) **VERFAHREN ZUR HERSTELLUNG VON N-(5-AMINO-2-CYANO-4-FLUOR-PHENYL)-SULFONAMIDEN**
METHOD FOR THE PRODUCTION OF N-(5-AMINO-2-CYANO-4-FLUORO-PHENYL)-SULPHONAMIDES
PROCEDE DE PRODUCTION DE N-(5-AMINO-2-CYANO-4-FLUOROPHENYL)-SULFONAMIDES

(30) Priorität: 24.07.1997 DE 19731783
(43) Veröffentlichungstag der Anmeldung: 31.05.2000
(73) Patentinhaber: Bayer CropScience AG, 40789 Monheim (DE)
(72) Erfinder: HUPPERTS, Achim, D-40217 Düsseldorf (DE); DREWES, Mark, Wilhelm, D-40764 Langenfeld (DE); ERDMAN, David, D-51061 Köln (DE); LANTZSCH, Reinhard, D-42115 Wuppertal (DE)
(86) Internationale Anmeldenummer: PCT/EP1998/004324
(87) Internationale Veröffentlichungsnummer: WO 1999/005098

(56) Entgegenhaltungen:
- EP-A- 0 648 772
- K. WALLENFELS, ET AL.: "1,3,5-Trichlor-2,4,6-tricyan-benzol und 1,3,5-Trifluor-2,4,6-tricyan-benzol" TETRAHEDRON, Bd. 23, Nr. 4, April 1967, Seiten 1845-1855, XP002085246 OXFORD, GB
- J. OHMORI, ET AL.: "6-(1H-Imidazol-1-yl)-7-nitro-2,3(1H,4H)-q uibaxalinedione hydrochloride (YM90K) and related compounds: structure-activity relationships for the AMPA-type non-NMDA receptor" JOURNAL OF MEDICINAL CHEMISTRY, Bd. 37, Nr. 4, 18. Februar 1994, Seiten 467-475, XP002020660 Washington, DC, US
- A.O. MILLER, ET AL.: "Reactions of polyfluoroarenes with hexamethyldisilazane and with 1,1,1-trimethyl-N,N-bis(trimethylsilyl) stannaneamine in the presence of caesium fluoride" JOURNAL OF FLUORINE CHEMISTRY, Bd. 75, Nr. 2, Dezember 1995, Seiten 169-172, XP004020479 Lausanne, CH
- K. NAKAMURA, ET AL.: "Studies on antiinflammarory agents II. Synthesis and pharmacological properties of 2'-(phenylthio)methanesulphonanilides and related derivatives" CHEMICAL AND PHARMACEUTICAL BULLETIN, Bd. 41, Nr. 5, Mai 1993, Seiten 894-906, XP000652372 Tokyo, JP

## Beschreibung

Die Erfindung betrifft ein neues Verfahren zur Herstellung von N-(5-Amino-2-cyano-4-fluor-phenyl)-sulfonamiden, welche als Zwischenprodukte bei der Herstellung von Herbiziden bekannt sind.

Es ist bekannt, daß man bestimmte N-(5-Amino-2-cyano-4-fluor-phenyl)-alkansulfonamide, wie z.B. N-(5-Amino-2-cyano-4-fluor-phenyl)-methansulfonamid, erhält, wenn man entsprechende halogenierte Benzolderivate, wie z.B. 1-Amino-4-cyano-2,5-difluor-benzol, mit Alkansulfonamiden, wie z.B. Methansulfonamid, in Gegenwart eines Säurebindemittels, wie z.B. Kaliumcarbonat, und in Gegenwart eines Verdünnungsmittels, wie z.B. N-Methyl-pyrrolidon, erhitzt (siehe EP-A-648772). Die gewünschten Produkte werden nach diesem Verfahren jedoch in unbefriedigenden Ausbeuten erhalten. Es besteht also Bedarf an einem günstigeren Herstellungsverfahren für N-(5-Amino-2-cyano-4-fluor-phenyl)-sulfonamide.

Es wurde nun gefunden, daß man N-(5-Amino-2-cyano-4-fluor-phenyl)-sulfonamide der allgemeinen Formel (I) 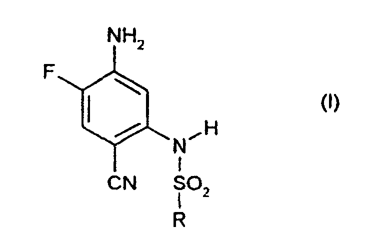 in welcher
- R: für jeweils gegebenenfalls durch Halogen substituiertes Alkyl, Alkenyl, Alkinyl mit jeweils bis zu 6 Kohlenstoffatomen, für jeweils gegebenenfalls durch Halogen oder C₁₋₄-Alkyl substitutiertes Cycloalkyl, Cycloalkylalkyl mit jeweils 3 bis 6 Kohlenstoffatomen in der Cycloalkylgruppe und gegebenenfalls 1 bis 4 Kohlenstoffatomen im Alkylteil steht,
in hohen Ausbeuten und in sehr guter Qualität erhält, wenn man in einem ersten Schritt 2-Amino-4,5-difluor-benzonitril der Formel (II) 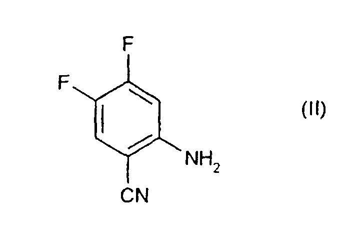 mit Sulfonsäurehalogeniden der allgemeinen Formel (III)

X-SO₂-R (III)

in welcher
- R: die vorstehend angegebene Bedeutung hat und
- X: für Fluor, Chlor oder Brom steht,
in Gegenwart eines Säureakzeptors und in Gegenwart eines Verdünnungsmittels bei Temperaturen zwischen 0°C und 150°C umsetzt
und die hierbei als Zwischenprodukte erhaltenen N-(2-Cyano-4,5-difluor-phenyl)-sulfonamide der allgemeinen Formel (IV) und/oder N-(2-Cyano-4,5-difluor-phenyl)-sulfonimide der allgemeinen Formel (V) 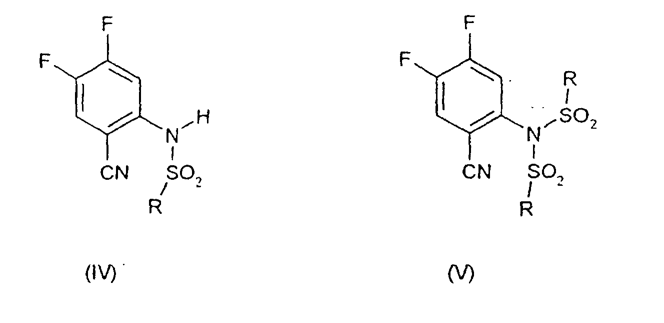 in welchen
- R: die vorstehend angegebene Bedeutung hat,
als reine Stoffe oder als Gemische in einem zweiten Schritt mit Ammoniak in Gegenwart eines Verdünnungsmittels bei Temperaturen zwischen 100° und 200°C umsetzt.

Überraschenderweise können nach dem erfindungsgemäßen Verfahren die N-(5-Amino-2-cyano-4-fluor-phenyl)-sulfonamide der allgemeinen Formel (I) auf relativ einfache Weise in hohen Ausbeuten und in sehr guter Qualität erhalten werden, wobei über ein Gemisch von Zwischenprodukten ein reines Endprodukt hergestellt werden kann. Die Zwischenprodukte der Formeln (IV) und (V) können als Gemische in praktisch quantitativer Ausbeute erhalten werden.

Vorteilhaft beim erfindungsgemäßen Verfahren ist vor allem, daß auf die Verwendung des relativ teuren 2,4,5-Trifluor-benzonitrils verzichtet werden kann, und der problematische Austausch eines Fluor-Substituenten gegen eine Sulfonylamino-Gruppierung entfällt.

Man erhält die neue Verbindung der Formel (II), wenn man 4,5-Difluor-2-nitrobenzonitril der Formel (VI) 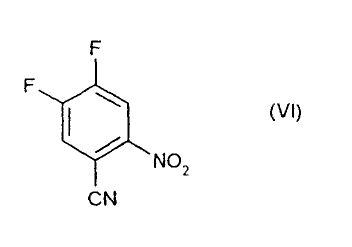 mit einem für die Umwandlung von aromatischen Nitroverbindungen in entsprechende Aminoverbindungen üblichen Reduktionsmittel, wie z.B. (a) Wasserstoff in Gegenwart eines Katalysators wie z.B. Platin oder Palladium (die beiden letztgenannten gegebenenfalls "vergiftet" und auf einem Trägermaterial, wie z.B. Aktivkohle oder Bariumsulfat), in Gegenwart eines Verdünnungsmittel, wie z.B. Tetrahydrofuran oder Dioxan, oder (b) Metalle oder Metallsalze, wie z.B. Zinn, Zinn(II)-chlorid, Eisen (-Pulver) in Gegenwart einer Säure, wie z.B. Salzsäure oder Essigsäure, und gegebenenfalls zusätzlich in Gegenwart eines Verdünnungsmittels, wie z.B. Methanol oder Ethanol, bei Temperaturen zwischen 0°C und 150°C, vorzugsweise zwischen 10°C und 100°C umsetzt (vgl. die Herstellungsbeispiele).

Das als Vorprodukt benötigte 4,5-Difluor-2-nitro-benzonitril der Formel (VI) ist bereits bekannt (siehe JP 07070041 - zitiert in Chem. Abstracts 123:111678). Gemäß der zitierten Patentliteratur kann 4,5-Difluor-2-nitro-benzonitril durch Umsetzung von 2-Brom-4,5-difluor-nitrobenzol mit Kupfer(1)-cyanid in N,N-Dimethyl-formamid hergestellt werden.

Man erhält die Verbindung der Formel (VI) aber auch, wenn man 3,4-Difluor-benzonitril mit Salpetersäure, gegebenenfalls in Gegenwart von Schwefelsäure, bei Temperaturen zwischen -10°C und +30°C umsetzt (vgl. die Herstellungsbeispiele).

Überraschenerweise verläuft diese Nitrierung sehr einheitlich (regioselektiv) und die unter den Nitrierungsbedingungen zu erwartende Hydrolyse der Cyanogruppe ist nur in sehr geringem Umfang festzustellen.

Die beim erfindungsgemäßen Verfahren zur Herstellung von N-(5-Amino-2-cyano-4-fluor-phenyl)-sulfonamiden der allgemeinen Formel (I) weiter als Ausgangsstoffe zu verwendenden Sulfonsäurehalogenide sind durch die Formel (III) allgemein definiert.

In den vorstehenden Formeln stehen vorzugsweise
- R: für jeweils gegebenenfalls durch Fluor oder Chlor substituiertes Methyl, Ethyl, n- oder i-Propyl, n-, i-, s- oder t-Butyl, Ethenyl, Propenyl, Butenyl, Ethinyl, Propinyl oder Butinyl, für jeweils gegebenenfalls durch Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl und
- X: für Chlor.

Die Ausgangsstoffe der Formel (III) sind bekannte Synthesechemikalien.

Das erfindungsgemäße Verfahren zur Herstellung von N-(5-Amino-2-cyano-4-fluorphenyl)-sulfonamiden der allgemeinen Formel (I) wird im ersten Schritt unter Verwendung eines Säureakzeptors durchgeführt. Es kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, - hydrogencarbonate, -hydride, -hydroxide oder -alkanolate, wie beispielsweise Natrium-, Kalium- oder Calcium-acetat, Lithium-, Natrium-, Kalium- oder Calciumamid, Natrium-, Kalium- oder Calcium-carbonat, Natrium-, Kalium- oder Calciumhydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Vorzugsweise werden basische organische Stickstoffverbindungen als Säureakzeptoren eingesetzt.

Als Verdünnungsmittel zur Durchführung des ersten Schrittes des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Vorzugsweise werden aprotisch polare organische Lösungsmittel, insbesondere Aceton oder Acetonitril, oder aber basiche organische Stickstoffverbindungen, wie Pyridin oder 5-Ethyl-2-methyl-pyridin, als Verdünnungsmittel eingesetzt.

Die Reaktionstemperaturen können bei der Durchführung des ersten Schrittes des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Der erste Schritt des erfindungsgemäßen Verfahrens wird im allgemeinen unter Normaldruck durchgeführt. Es ist jedoch auch möglich, das erfindungsgemäße Verfahren unter erhöhtem oder vermindertem Druck - im allgemeinen zwischen 0.1 bar und 10 bar - durchzuführen.

Zur Durchführung des ersten Schrittes des erfindungsgemäßen Verfahren setzt man auf 1 Mol 2-Amino-4,5-difluor-benzonitril der formel (II) im allgemeinen zwischen 1 Mol und 10 Mol, vorzugsweise zwischen 2 Mol und 5 Mol, Sulfonsäurehalogenid der allgemeinen Formel (III) und zwischen 1 Mol und 10 Mol, vorzugsweise zwischen 2 Mol und 5 Mol Säureakzeptor, ein.

In einer bevorzugten Ausführungsform des ersten Schrittes des erfindungsgemäßen Verfahrens wird das 2-Amino-4,5-difluor-benzonitril der Formel (II) zusammen mit einem Säureakzeptor und einem Verdünnungsmittel vorgelegt und das Sulfonsäurehalogenid der allgemeinen Formel (III) wird dann unter Rühren - und gegebenenfalls unter Kühlen - langsam in diese Mischung eindosiert. Die komplette Reaktionsmischung wird dann - gegebenenfalls bei erhöhter Temperatur - bis zum Ende der Umsetzung gerührt.

Die Aufarbeitung der Gemische der Zwischenprodukte der Formeln (IV) und (V) kann auf übliche Weise durchgeführt werden. Beispielsweise wird mit Wasser oder einer verdünnten wässrigen Säure verrührt, die organische Phase abgetrennt, die wässrige Phase gegebenenfalls mit einem mit Wasser praktisch nicht mischbaren organischen Lösungsmittel, wie z.B. Essigsäureethylester, nachextrahiert, die vereinigten organischen Phasen werden getrocknet und filtriert. Zur Isolierung des Zwischenproduktgemisches wird vom Filtrat das Lösungsmittel unter vermindertem Druck sorgfältig abdestilliert.

Die so erhaltenen Gemische der Zwischenprodukte der Formeln (IV) und (V) können vorteilhaft ohne weitere Reinigung zur Umsetzung gemäß dem zweiten Schritt des erfindungsgemäßen Verfahrens eingesetzt werden.

Der zweite Schritt des erfindungsgemäßen Verfahrens wird vorzugsweise unter Verwendung eines Verdünnungsmittels durchgeführt. Als Verdünnungsmittel kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, t-Butyl-methylether, t-Pentyl-methylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Vorzugsweise werden aprotisch polare organische Lösungsmittel, insbesondere Diisopropylether, t-Butyl-methylether, t-Pentyl-methylether, Tetrahydrofuran, Dioxan, Ethylenglykol-dimethylether oder -diethylether als Verdünnungsmittel eingesetzt

Die Reaktionstemperaturen können bei der Durchführung des zweiten Schrittes des erfindungsgemäßen Verfahrens in einem größeren Bereich variiert werden. Im allgemeinen arbeitet man bei Temperaturen zwischen 50°C und 200°C, vorzugsweise zwischen 100°C und 180°C.

Der zweite Schritt des erfindungsgemäßen Verfahrens wird im allgemeinen in einem geschlossenen Reaktionsgefaß (insbesondere im Autoklaven) unter erhöhtem Druck durchgetührt, wobei der Druck von der eingestellten Temperatur und vom verwendeten Lösungsmittel abhängt.

Zur Durchführung des zweiten Schrittes des erfindungsgemäßen Verfahrens setzt man auf 1 Mol der Summe aus den Zwischenprodukten der Formeln (IV) und (V) im allgemeinen zwischen 1 und 100 Mol, vorzugsweise zwischen 5 und 50 Mol Ammoniak ein.

In einer bevorzugten Ausführungsform des zweiten Schrittes des erfindungsgemäßen Verfahrens werden die Reaktionskomponenten der Formel (IV) und/oder (V) mit Ammoniak und einem Verdünnungsmittel bei Raumtemperatur (ca. 20°C) vermischt und in einem geschlossenen Reaktionsgefaß so lange erhitzt, bis die Umsetzung abgeschlossen ist.

Die Aufarbeitung und Isolierung der Produkte der Formel (t) kann nach üblichen Methoden erfolgen. Beispielsweise wird das Reaktionsgemisch nach dem Abkühlen filtriert und vom Filtrat das Lösungsmittel unter vermindertem Druck sorgfältig abdestilliert. Das Produkt kann auf diese Weise als Rückstand im allgemeinen in guter Qualität erhalten werden.

Die nach dem erfindungsgemäßen Verfahren herzustellenden Verbindungen der Formel (I) können als Zwischenprodukte zur Herstellung von herbizid wirksamen Verbindungen verwendet werden (siehe EP-A-648749, EP-A-648772, WO-A-95/29158).

Die Zwischenprodukte der Formeln (IV) und (V) können ebenfalls als Vorstufen für die Herstellung von Herbiziden eingesetzt werden (siehe EP-A 609734).

### Herstellungsbeispiele:

### Beispiel 1

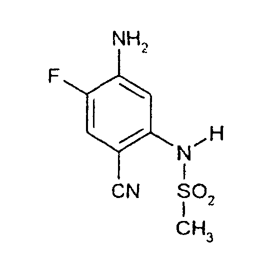

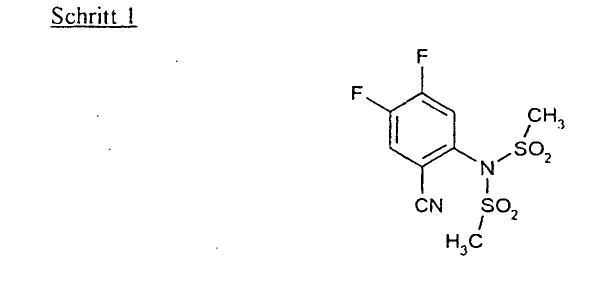

15 g (130 mMol) Methansulfonsäurechlorid werden tropfenweise unter Rühren zu einer Mischung aus 3,7 g (24 rnMol) 2-Amino-4,5-difluor-benzonitril, 5,0 g (33 mMol) 1,8 Diazabicyclo[5,4,0]-undec-7-en (DBU) und 50 ml Pyridin gegeben und die Reaktionsmischung wird dann etwa eine Stunde lang bei 50°C gerührt. Anschließend wird im Wasserstrahlvakuum eingeengt, der Rückstand mit 100 ml 20%iger Salzsäure und 10 ml Essigsäureethylester verrührt und das kristallin angefallene Produkt durch Absaugen isoliert.

Man erhält 3,3 g (44% der Theorie) 4,5-Difluor-2-(bis-methansulfonyl-amino)-benzonitril vom Schmelzpunkt 144°C.

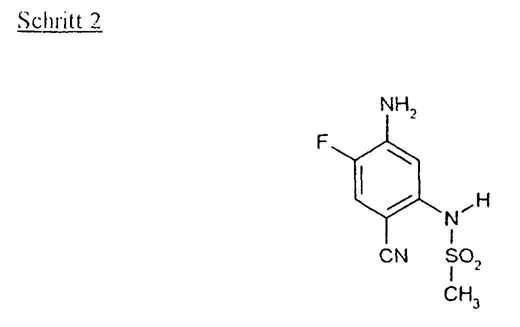

In einem 100ml-Autoklaven werden 2 ml Ammoniak einkondensiert und 2,0 g (6.4 mMol) 4,5-Difluor-2-(bis-methansulfonyl-amino)-benzonitril sowie 40 ml Tetrahydrofuran dazu gegeben. Das Reaktionsgemisch wird dann im verschlossenen Autoklaven 15 Stunden lang auf 150°C erhitzt. Nach Abkühlen wird filtriert und das Filtrat im Wasserstrahlvakuum eingeengt. Nach Waschen mit 2N-Salzsäure und mit Wasser erhält man 0,50 g (34% der Theorie) N-(5-Amino-2-cyano-4-fluor-phenyl)-methansulfonamid vom Schmelzpunkt 235°C.

### Beispiel 2

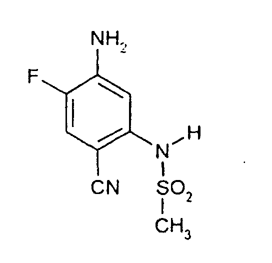

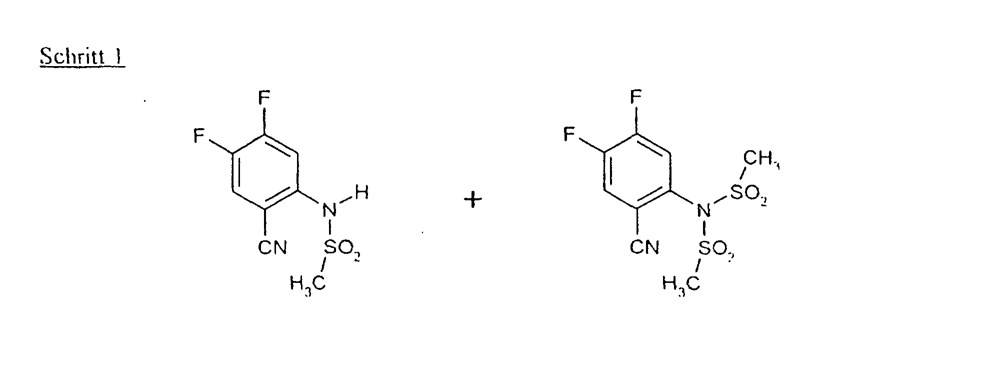

4,6 g (40 mMol) Methansulfonsäurechlorid werden tropfenweise unter Rühren zu einer Mischung aus 1,54 g (10 mMol) 2-Amino-4,5-difluor-benzonitril, 4,0 g (40 mMol) Triethylamin und 50 ml Acetonitril gegeben und die komplette Reaktionsmischung wird dann etwa eine Stunde lang unter Rückfluß erhitzt. Nach dem Abkühlen wird 30 Minuten lang mit 100 ml Eiswasser verrührt und nach Trennen der Phasen die wässrige Phase mit 50 ml Essigsäureethylester nachextrahiert. Die vereinigten organischen Phasen werden mit gesättigter wässriger Natriumhydrogencarbonat-Lösung und dann mit Wasser gewaschen, mit Natriumsulfat getrocknet und filtriert. Vom Filtrat wird das Lösungsmittel im Wasserstrahlvakuum sorgfältig abdestilliert.

Man erhält als Rückstand 2,9 g einer Mischung aus 43% 4,5-Difluor-2-(bis-methylsulfonylamino)-benzonitril und 57% 4,5-Difluor-2-methylsulfonylamino-benzonitril (nach GC/MS), was einer quantitativen Gesamt-Ausbeute entspricht.

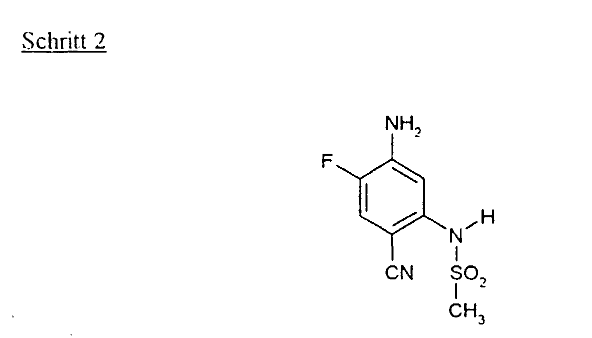

In einem 100ml-Autoklaven werden 4,5 ml Ammoniak einkondensiert und das gemäß Stufe 2 erhaltene Produktgemisch (2,9 g) sowie 50 ml Tetrahydrofuran dazu gegeben. Das Reaktionsgemisch wird dann im verschlossenen Autoklaven 15 Stunden lang auf 150°C erhitzt. Nach Abkühlen wird filtriert und das Filtrat im Wasserstrahlvakuum eingeengt. Nach Waschen mit 2N-Salzsäure und mit Wasser erhält man 1,0 g (44% der Theorie) N-(5-Amino-2-cyano-4-fluor-phenyl)-methansulfonamid vom Schmelzpunkt 235°C.

### Beispiel 3

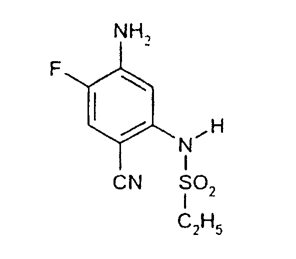

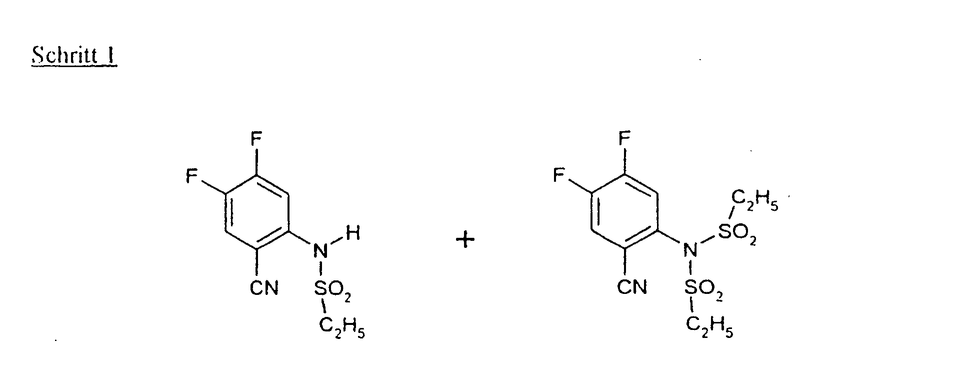

5,1 g (40 mMol) Ethansulfonsäurechlorid werden tropfenweise unter Rühren zu einer Mischung aus 1,54 g (10 mMol) 2-Amino-4,5-difluor-benzonitril, 4,0 g (40 mMol) Triethylamin und 50 ml Acetonitril gegeben und die komplette Reaktionsmischung wird dann etwa zwei Stunden lang unter Rückfluß erhitzt. Nach dem Abkühfen wird 30 Minuten lang mit 100 ml Eiswasser verrührt und nach Trennen der Phasen die wässrige Phase noch mit 50 ml Essigsäureethylester nachextrahiert. Die vereinigten organischen Phasen werden mit Natriumsulfat getrocknet und filtriert. Vom Filtrat wird das Lösungsmittel im Wasserstrahlvakuum sorgfältig abdestilliert.

Man erhält als Rückstand 3,0 g einer Mischung aus 67% 4,5-Difluor-2-(bis-ethylsulfonylamino)-benzonitril und 33% 4,5-Difluor-2-ethylsulfonylamino-benzonitril (nach GC/MS), was einer quantitativen Gesamt-Ausbeute entspricht.

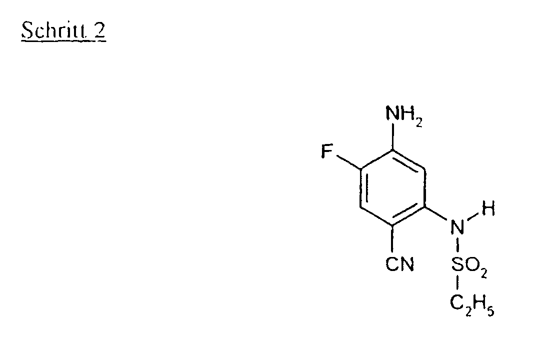

In einem 100ml-Autoklaven werden 4 ml Ammoniak einkondensiert und 3,0 g des Produktgemisches aus Stufe 1 sowie 50 ml Tetrahydrofuran dazu gegeben. Das Reaktionsgemisch wird im geschlossenen Autoklaven 15 Stunden auf 150°C erhitzt und nach dem Abkühlen filtriert. Das Filtrat wird im Wasserstrahlvakuum eingeengt, mit 2N-Salzsäure und mit Wasser gewaschen und getrocknet.

Man erhält 1,1 g (45% der Theorie eines 95,5%igen Produktes) 4-Amino-5-fluor-2-ethylsulfonylamino-benzonitril vom Schmelzpunkt 170°C.

### Ausgangverbindung der Formel (II)

### Beispiel (II-1)

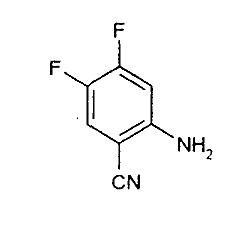

3,68 g (20 mMol) 4,5-Difluor-2-nitro-benzonitril werden in 40 ml Dioxan gelöst und 300 mg Platin auf Kohle (5%) dazu gegeben. Anschließend wird die Suspension bei 20°C bis 25°C solange unter Wasserstoff gerührt, bis 1,45 Liter Wasserstoff aufgenommen worden sind. Die Mischung wird dann über Kieselgel filtriert und das Filtrat im Wasserstrahlvakuum eingeengt. Der Rückstand wird dann säulenchromatografisch (Kieselgel, Hexan/Essigsäureethylester) aufgearbeitet.

Man erhält 2,19 g (72% der Theorie) 2-Amino-4,5-difluor-benzonitril vom Schmelzpunkt 114°C und aus einer weiteren Fraktion 0,52 g (15% der Theorie) 2-Amino-4,5-difluor-benzamid.

### Beispiel (II-2)

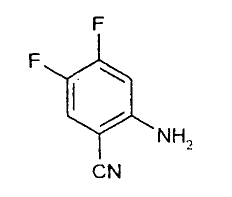

11,0 g (59 mMol) 4,5-Difluor-2-nitro-benzonitril werden in 175 ml Essigsäure ("Eisessig") gelöst und 20 g (358 mMol) Eisen (-Pulver) werden portionsweise dazu gegeben. Die Reaktionstemperatur wird dabei durch Kühlen mit einem Wasserbad bei 40°C bis 50°C gehalten. Das komplette Reaktionsgemisch wird dann noch 3 Stunden lang bei 50°C gerührt. Nach Abkühlen auf Raumtemperatur wird die Mischung auf 200 ml Eiswasser gegeben. Man extrahiert zweimal mit je 50 ml Essigsäureethylester, vereinigt, die organischen Extraktionslösungen, wäscht, mit gesättigter Natriumhydrogencarbonat-Lösung und dann mit Wasser, trocknet mit Natriumsulfat und filtriert. Das Filtrat wird im Wasserstrahlvakuum eingeengt und der Rückstand säulenchromatografisch (Kieselgel, Hexan/Essigsäureethylester) aufgearbeitet.

Man erhält 7,7 g (85% der Theorie) 2-Amino-4,5-difluor-benzonitril vom Schmelzpunkt 114°C.

### Ausgangsverbindung der Formel (VI):

### Beispiel (VI-1)

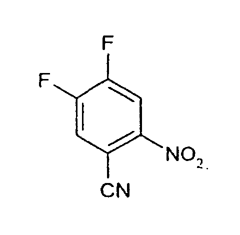

Eine Mischung aus 40 ml Schwefelsäure (97%ig) und 30 ml Salpetersäure (98%ig) wird auf 0°C abgekühlt. Dann werden 13,9 g (0,10 Mol) 3,4-Difluor-benzonitril portionsweise so dazu gegeben, daß die Reaktionstemperatur unter 5°C bleibt. Die komplette Reaktionsmischung wird 5 Stunden bei 5°C bis 10°C gerührt und nach Erwärmen auf 20°C weitere 2 Stunden gerührt. Anschließend wird die Mischung auf 400 g Eis gegeben, das kristallin angefallene Produkt durch Absaugen isoliert und in 20 ml Methylenchlorid aufgenommen. Die wässrige Phase wird zweimal mit je 30 ml Methylenchlorid nachextrahiert. Die organischen Phasen werden vereinigt. mit gesättigter Natriumhydrogencarbonat-Lösung und mit Wasser gewaschen, mit Natriumsulfat getrocknet und filtriert. Vom Filtrat wird das Lösungsmittel im Wasserstrahlvakuum sorgfältig abdestilliert.

Man erhält 10,2 g (55% der Theorie) 4,5-Difluor-2-nitro-benzonitril vom Schmelzpunkt 75°C.

## Patentansprüche

1. Verfahren zum Herstellen von N-(5-Amino-2-cyano-4-fluor-phenyl)-sulfonamiden der allgemeinen Formel (I), 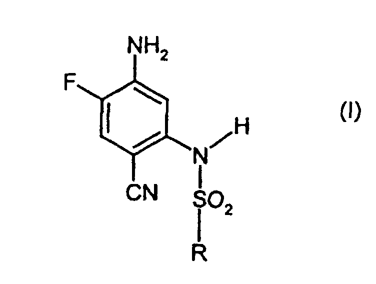 in welcher
R für jeweils gegebenenfalls durch Halogen substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil steht,
**gekennzeichnet durch** die folgenden Schritte:
A) Umsetzen von 2-Amino-4,5-difluor-benzonitril der Formel (II) 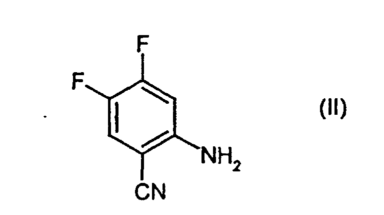 mit Sulfonsäurehalogeniden der allgemeinen Formel (III),
X-SO₂-R (III)
in welcher
R die vorstehend angegebene Bedeutung hat und
X für Fluor, Chlor oder Brom steht,
in Gegenwart eines Säureakzeptors und in Gegenwart eines Verdünnungsmittels bei Temperaturen zwischen 0°C und 150°C und
B) Umsetzen der im Schritt A) erhaltenen N-(2-Cyano-4,5-difluorphenyl)-sulfonamide der allgemeinen Formel (IV) und/oder N-(2-Cyano-4,5-difluor-phenyl)-sulfonimide der allgemeinen Formel (V), 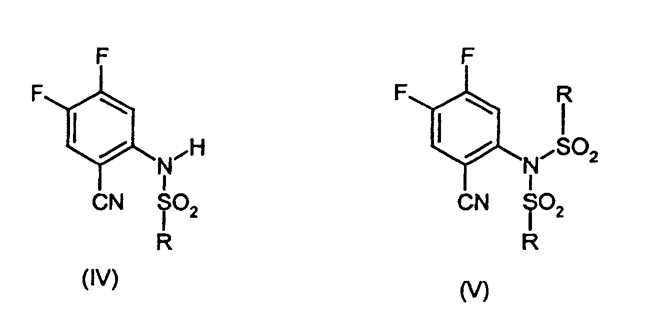
in welchen
R die vorstehend angegebene Bedeutung hat,
als reine Stoffe oder als Gemische mit Ammoniak in Gegenwart eines Verdünnungsmittels bei Temperaturen zwischen 100° und 200°C.

2. Verfahren nach Anspruch 1, **dadurch gekennzeichnet, dass**
R für jeweils gegebenenfalls durch Fluor oder Chlor substituiertes Methyl, Ethyl, n- oder i-Propyl, n-, i-, s- oder t-Butyl, Ethenyl, Propenyl, Butenyl, Ethinyl, Propinyl oder Butinyl, für jeweils gegebenenfalls durch Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl und
X für Chlor steht.

3. Verfahren nach Anspruch 1 oder 2, **dadurch gekennzeichnet, dass** als Säureakzeptor basische organische Stickstoffverbindungen eingesetzt werden.

4. Verfahren nach einem der Ansprüche 1 bis 3, **dadurch gekennzeichnet, dass** als Verdünnungsmittel im Schritt A) Aceton und/oder Acetonitril und im Schritt B) Pyridin und/oder 5-Ethyl-2-methyl-pyridin eingesetzt werden.

## Claims

1. Process for preparing N-(5-amino-2-cyano-4-fluoro-phenyl)-sulphonamides of the general formula (I) 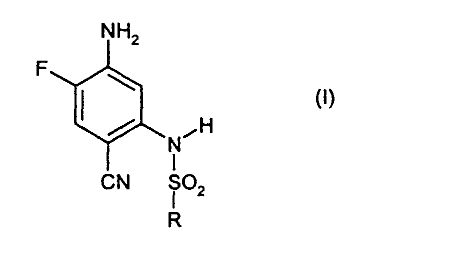 in which
R represents in each case optionally halogen-substituted alkyl, alkenyl or alkinyl having in each case up to 6 carbon atoms, represents in each case optionally halogen- or C₁-C₄-alkyl-substituted cycloalkyl or cycloalkylalkyl having in each case 3 to 6 carbon atoms in the cycloalkyl group and, if appropriate, 1 to 4 carbon atoms in the alkyl moiety,
**characterized by** the following steps:
A) reaction of 2-amino-4,5-difluoro-benzonitrile of the formula (II) 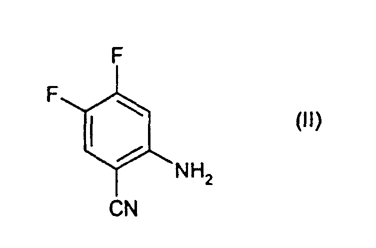 with sulphonyl halides of the general formula (III)
X-SO₂-R (III)
in which
R is as defined above and
X represents fluorine, chlorine or bromine,
in the presence of an acid acceptor and in the presence of a diluent at temperatures between 0°C and 150°C and
B) reaction of the N-(2-cyano-4,5-difluoro-phenyl)sulphonamides of the general formula (IV) and/or N-(2-cyano-4,5-difluoro-phenyl)-sulphonimides of the general formula (V) obtained in step A) 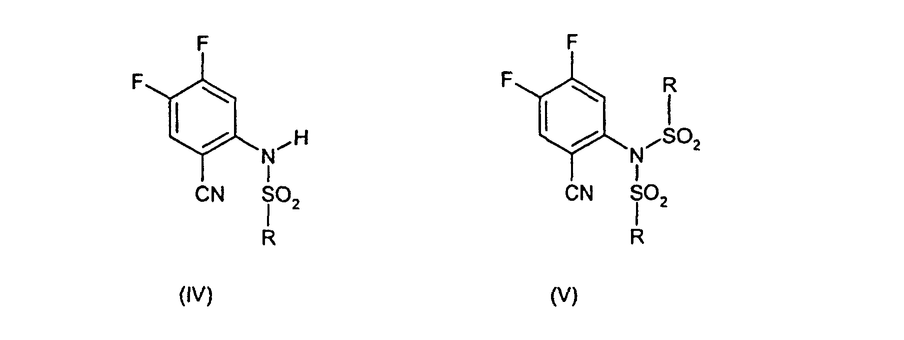
in which
R is as defined above
as pure substances or as mixtures with ammonia in the presence of a diluent at temperatures between 100° and 200°C.

2. Process according to Claim 1, **characterized in that**
R represents in each case optionally fluorine- or chlorine-substituted methyl, ethyl, n- or i-propyl, n-, i-, s- or t-butyl, ethenyl, propenyl, butenyl, ethinyl, propinyl or butinyl, represents in each case optionally fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, and
X represents chlorine.

3. Process according to Claim 1 or 2, **characterized in that** the acid acceptors used are basic organic nitrogen compounds.

4. Process according to any of Claims 1 to 3, **characterized in that** the diluent used in step A) is acetone and/or acetonitrile and the diluent used in step B) is pyridine and/or 5-ethyl-2-methyl-pyridine.

## Revendications

1. Procédé de production de N-(5-amino-2-cyano-4-fluorcphényl)-sulfonamides de formule générale (I), 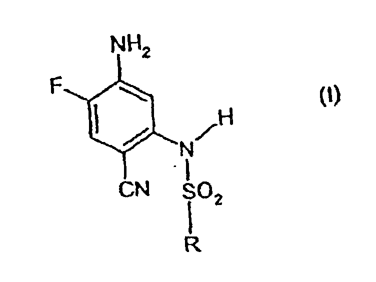 dans laquelle
R est un reste alkyle, un reste alcényle ou un reste alcynyle ayant chacun jusqu'à 6 atomes de carbone et chacun étant éventuellement substitué par un halogène, un reste cycloalkyle ou un reste cycloalkyle-alkyle ayant chacun 3 à 6 atomes de carbone dans le groupe cycloalkyle et le cas échéant 1 à 4 atomes de carbone dans la partie alkyle et chacun étant éventuellement substitué par un halogène ou un radical alkyle en C₁ à C₄,
**caractérisé par** les étapes suivantes :
A) réaction d'un 2-amino-4,5-difluoro-benzonitrile de formule (II) 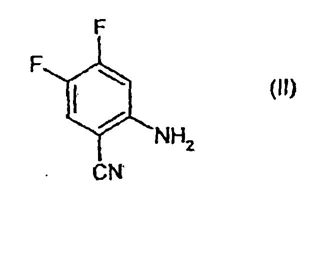 avec des halogénures d'acide sulfonique de formule générale (III),
X-SO₂-R (III)
dans laquelle
R a la définition indiquée ci-dessus et
X représente le fluer, le chlore ou le brome,
en présence d'un accepteur d'acide et en présence d'un diluant, à des températures comprises entre 0°C et 150°C, et
B) réaction des N-(2-cyano-4,5-difluorophényl)-sulfonamide de formule générale (IV) et/ou des N-(2-cyano-4,5-difluorophényl)-sulfonimide de formule générale (V) obtenus dans l'étape A), 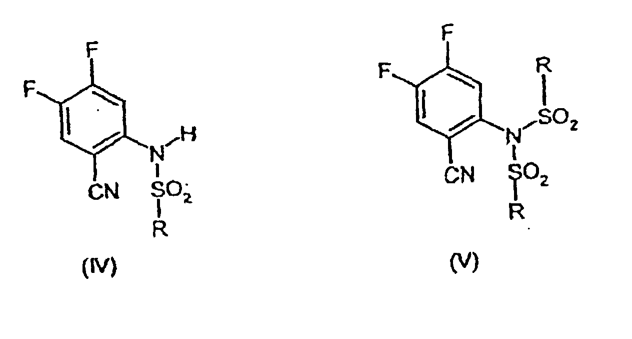 formules dans lesquelles
R a la définition indiquée ci-dessus,
à l'état de substances pures ou de mélanges avec l'ammoniac, en présence d'un diluant, à des températures comprises entre 100 et 200°C.

2. Procédé suivant la revendication 1, **caractérisé en ce que**
R désigne un reste méthyle, éthyle, n-propyle, isopropyle, n-butyle, isobutyle, sec.-butyle, tertiobutyle, éthényle, propényle, butényle, éthynyle, propynyle ou butynyle dont chacun est éventuellement substitué par du fluer ou du chlore, un reste cyclopropyle, cyclobutyle, cyclopentyle, cyclohexyle, cyclopropylméthyle, cyclobutylméthyle, cyclopentylméthyle ou cyclohexylméthyle dont chacun est éventuellement substitué par du fluor, du chlore, un radical méthyle ou éthyle, et
X représente le chlore.

3. Procédé suivant la revendication 1 ou 2, **caractérisé en ce qu'**on utilise comme accepteur d'acide des bases organiques azotées.

4. Procédé suivant l'une des revendications 1 à 3, **caractérisé en ce qu'**on utilise comme diluant dans l'étape A) l'acétone et/ou l'acétonitrile et dans l'étape B) la pyridine et/ou la 5-éthyl-2-méthyl-pyridine.
